## Europäisches Patentamt
## European Patent Office
## Office européen des brevets

(19)

(11) Veröffentlichungsnummer: **0 152 029**
**B1**

(12)

# EUROPÄISCHE PATENTSCHRIFT

(45) Veröffentlichungstag der Patentschrift:
**18.10.89**

(51) Int. Cl.⁴: **A 61 M 1/00**

(21) Anmeldenummer: **85101057.9**

(22) Anmeldetag: **01.02.85**

(54) Verfahren zur Herstellung einer Saugflasche zum Absaugen von Sekreten aus Wundhöhlen.

(30) Priorität: **08.02.84 DE 3404382**

(43) Veröffentlichungstag der Anmeldung:
**21.08.85 Patentblatt 85/34**

(45) Bekanntmachung des Hinweises auf die Patenterteilung:
**18.10.89 Patentblatt 89/42**

(84) Benannte Vertragsstaaten:
**AT CH DE FR GB IT LI NL SE**

(56) Entgegenhaltungen:
**EP-A- 0 061 723**
**EP-A- 0 066 699**
**DE-A- 1 810 801**
**DE-B- 2 820 517**
**DE-B- 2 826 650**

**Die Akte enthält technische Angaben, die nach dem Eingang der Anmeldung eingereicht wurden und die nicht in dieser Patentschrift enthalten sind.**

(73) Patentinhaber: **Härle, Anton, Prof. Dr., Drechslerweg 40, D-4400 Münster-Roxel (DE)**

(72) Erfinder: **Härle, Anton, Prof. Dr., Drechslerweg 40, D-4400 Münster-Roxel (DE)**

(74) Vertreter: **Habbel, Hans-Georg, Dipl.-Ing., Postfach 3429 Am Kanonengraben 11, D-4400 Münster (DE)**

## Beschreibung

Die Erfindung bezieht sich auf ein Verfahren zur Herstellung einer Saugflasche gemäß dem Oberbegriff des Hauptanspruches.

Saugflaschen werden temporär postoperativ nach chirurgischen Eingriffen zum Absaugen von Wundsekreten und zur Adaption der Wundflächen über einen Applikationszeitraum von beispielsweise 3 Tagen eingesetzt. Die Saugflasche ist dabei als ein starrwandiger Behälter mit vorbereitetem negativem Druck ausgebildet und ist vorzugsweise für den Einmalgebrauch bestimmt und wird vorwiegend an Wund-Drainagen und/oder an Saug-Spüldrainagen angeschlossen. Saugflaschen der gattungsbildenden Art sind in der DE-A-2 802 517 oder in der EP-A-0 061 723 beschrieben und weisen dabei einen Druckanzeiger auf, der vornehmlich im Kopfbereich der Saugflasche angeordnet ist und die Aufgabe hat, den gebrauchsfertigen Zustand der Saugflasche anzuzeigen, d.h. anzuzeigen, daß ein Unterdruck in der Saugflasche vorhanden ist und somit die Saugkapazität zur Verfügung gestellt wird.

Die bekannten Unterdruckanzeiger arbeiten dabei beispielsweise mit einem Balgprinzip, einer verformbaren elastischen Platte oder mit einem verschiebbaren von außen sichtbaren Kolben. Diese Unterdruckanzeiger haben den Nachteil, daß sie aufgrund der in der Saugflasche auftretenden Kondensation oder aufgrund von Verschmutzungen klemmen und festkleben können und daß sie vor allen Dingen nicht den in der Saugflasche tatsächlich herrschenden Unterdruck anzeigen, sondern nur anzeigen, daß ein Unterdruck vorhanden ist.

Die bekannten Saugflaschen mit den bekannten Druckanzeigern lassen also keine sichere Kontrolle oder Messung des Unterdruckes zu.

Das Aufnahmevermögen einer gegebenen Saugflasche hängt ausschließlich von dem in ihr herrschenden Unterdruck oder negativen Druck ab. Ist dieser Unterdruck erschöpft, ist das Saugvermögen der Saugflasche nicht mehr gegeben. Damit ist ein Ablaufen des Wundsekretes nicht mehr möglich und es kommt zum Sekretstau in der Wunde mit Aufweitung der Wundhöhle (Hämatom- oder Serombildung). Die Adaption der Wundränder ist aufgehoben und es kann leicht zu einer rückläufigen Infektion der Wunde kommen.

Durch die bekannten Druckanzeiger an den Saugflaschen kann also von außen das Pflegepersonal nicht das maximal mögliche Füllvolumen der Saugflasche bestimmen, das immer nur ein bestimmter Prozentsatz des Flaschenrauminhaltes sein kann.

Für die Beurteilung der Funktionsfähigkeit einer Wundsaug-Drainage ist es wesentlich, die Aufnahmekapazität bei einem bestimmten, primär angelegten Unterdruck zu kennen. Das Ziel des Verfahrens ist, mit Hilfe des Unterdrucks die Wundflächen aneinander zu pressen, die Förderung des Wundsekretes in Richtung Saugflasche zu bewirken und die Gefahr rückläufiger Sekretbewegungen auszuschalten, wenn die Saugflasche höher liegt als das Wundniveau, was bei der Lagerung und Pflege des Patienten nie ausgeschlossen werden kann.

Erstrebenswert ist daher ein Indikator, der in Abhängigkeit des in der Flasche fabrikationstechnisch hergestellten Unterdrucks aufzeigt, welches Füllvolumen die Flasche bei noch bestehendem Restunterdruck besitzt. So konnte es bei den bekannten Anordnungen nicht ausgeschlossen werden, daß eine gegebene Aufnahmekapazität der Flasche vorgetäuscht wurde, wenn diese z.B. nur zu 50% oder sogar weniger gefüllt war. In Wirklichkeit hatten solche Flaschen gar keine Aufnahmekapazität mehr, nämlich dann, wenn der vorgegebene fabrikationstechnisch eingestellte Unterdruck nicht mehr als 0,5 bar oder sogar weniger betrug.

Der Erfindung liegt die Aufgabe zugrunde, ein Verfahren zur Herstellung einer Saugflasche mit definiertem Unterdruckbereich zu schaffen, bei der das in Abhängigkeit des Unterdrucks stehende maximale Füllvolumen der Saugflasche von außen sichtbar und kontrollierbar ist.

Diese der Erfindung zugrundeliegende Aufgabe wird durch die Lehre des Hauptanspruches gelöst. Vorteilhafte Ausgestaltungen sind in den Unteransprüchen erläutert.

Nach der erfindungsgemäßen Verfahrensweise wird der Nachteil der bekannten Anordnungen dadurch vermieden, daß von außen erkennbar die Flasche mit einer Markierung versehen wird, die farbig oder durch Formung hergestellt werden kann. Diese Markierung zeigt das Füllvolumen an, das diese Flasche in Abhängigkeit des in ihr gezogenen Unterdrucks aufweist.

Vorteilhaft ist, daß die Flaschen mit einem Unterdruck ausgerüstet werden, der zwischen 0,8 und 1 bar liegt.

Vorzugsweise liegt der vom Hersteller einzustellende Unterdruck bei 0,85–0,95 bar.

Durch den erfindungsgemäßen Vorschlag wird somit sichergestellt, daß von außen erkennbar ist, unabhängig wie groß das Flaschenfüllvolumen ist, daß der die Wirkung der Saugflasche bedingende Unterdruck erschöpft oder bald erschöpft ist und daß es nunmehr erforderlich ist, die Saugflasche zu wechseln, um damit die der Saugflasche zugedachte Aufgabe zu erfüllen.

Der vorstehend erläuterte hohe Unterdruck hat mehrere bedeutungsvolle Funktionen:

a) Durch den hohen Unterdruck können 80–95% des Flaschenrauminhaltes befüllt werden,

b) die maximale Befüllbarkeit entspricht den Erwartungswerten und die Flasche hat so lange Unterdruck, bis sie wirklich voll ist,

c) der Unterdruck bleibt praktisch unverändert während des Befüllens, wodurch eine gleichbleibende Adaption der Wundränder bewirkt wird.

Beispiele für die maximale Befüllbarkeit einer Flasche sind nachfolgend aufgeführt:
0,3 bar Unterdruck – 30% Befüllbarkeit
0,5 bar Unterdruck – 50% Befüllbarkeit
0,9 bar Unterdruck – 90% Befüllbarkeit.

## Patentansprüche

1. Verfahren zur Herstellung einer Saugflasche zum Absaugen von Sekreten aus Wundhöhlen mit Evakuieren des Flascheninneren auf einen vorbestimmten Unterdruck, wobei die Saugflasche eine Vorrichtung zum Anzeigen des momentan herrschenden Unterdruckes sowie Anschlüsse für einen Saugschlauch aufweist, dadurch gekennzeichnet, daß an der Saugflasche eine von außen erkennbare Markierung in einer Höhe angebracht wird, die der Höhe des mit dem vorbestimmten Unterdruck in die Flasche einziehbaren Sekretvolumens entspricht.

2. Verfahren nach Anspruch 1, dadurch gekennzeichnet, daß die Markierung in einer Höhe angebracht ist, bei dem eine retrograde Sekretbewegung noch ausgeschlossen ist, wenn die Saugflasche nicht mehr als 1 m über das Wundniveau gehoben wird.

3. Verfahren nach Anspruch 1 oder 2, dadurch gekennzeichnet, daß die Markierung als Prozentangabe oder in ml-Angabe auf der Flasche vorgesehen wird.

4. Verfahren nach Anspruch 1 oder 2, dadurch gekennzeichnet, daß die Markierung durch eine an der Flaschenwandung vorgesehene farbige Kennzeichnung gebildet wird.

5. Verfahren nach Anspruch 1 oder 2, dadurch gekennzeichnet, daß die Markierung durch eine in der Flaschenwandung vorgesehene Formmarkierung gebildet wird.

6. Verfahren nach einem oder mehreren der vorhergehenden Ansprüche 1 bis 5, dadurch gekennzeichnet, daß der im Flascheninneren herrschende Unterdruck zwischen 0,8 und 1 bar eingestellt wird.

7. Verfahren nach Anspruch 6, dadurch gekennzeichnet, daß der im Flascheninneren eingestellte Unterdruck vorzugsweise bei 0,85–0,95 bar eingestellt wird.

## Claims

1. Method of constructing a suction bottle for the drawing off of secretions from wound cavities, with evacuation of the bottle interior to a predetermined negative pressure, the suction bottle comprising a means for indicating the instantaneously prevailing negative pressure and also connections for a suction hose, characterised in that a marking visible from the exterior is arranged on the suction bottle at a height which corresponds to the level of the volume of secretion which can be drawn into the bottle with the predetermined negative pressure.

2. Method according to claim 1, characterised in that the marking is arranged at a height at which a retrograde movement of secretion is still precluded if the suction bottle is not lifted more than 1 m above the wound level.

3. Method according to claim 1 or 2, characterised in that the marking is provided as a percentage indication, or an ml indication, on the bottle.

4. Method according to claim 1 or 2, characterised in that the marking is constituted by a coloured mark provided on the bottle wall.

5. Method according to claim 1 or 2, characterised in that the marking is constituted by a shape marking formed in the bottle wall.

6. Method according to one or more of the preceding claims 1 to 5, characterised in that the negative pressure prevailing in the bottle interior is set to between 0.8 and 1 bar.

7. Method according to claim 6, characterised in that the negative pressure set up in the bottle interior is set preferably to 0.85–0.95 bar.

## Revendications

1. Procédé de fabrication d'un flacon aspirateur destiné à aspirer les sécrétions de cavités de plaies avec mise sous vide du volume intérieur du flacon à une dépression prédéterminée, le flacon aspirateur présentant un dispositif destiné à indiquer la dépression régnant instantanément, ainsi que des raccords pour raccorder un tuyau d'aspiration, caractérisé en ce que, sur le flacon aspirateur, est formé un marquage lisible de l'extérieur, à un niveau qui correspond au niveau du volume de sécrétions qui peut être aspiré dans le flacon avec la dépression prédéterminée.

2. Procédé selon la revendication 1, caractérisé en ce que le marquage est prévu à une hauteur à laquelle un écoulement de retour des sécrétions est encore exclu lorsque le flacon aspirateur est élevé à pas plus de 1 m au-dessus du niveau de la plaie.

3. Procédé selon la revendication 1 ou 2, caractérisé en ce que le marquage est prévu sous la forme d'une indication de pourcentage ou d'une indication en ml sur le flacon.

4. Procédé selon la revendication 1 ou 2, caractérisé en ce que le marquage est formé par un repère coloré prévu sur la paroi du flacon.

5. Procédé selon la revendication 1 ou 2, caractérisé en ce que le marquage est formé par un marquage moulé prévu dans la paroi du flacon.

6. Procédé selon une ou plusieurs des revendications 1 à 5, caractérisé en ce que la dépression qui règne dans le volume intérieur du flacon est réglée à une valeur entre 0,8 et 1 bar.

7. Procédé selon la revendication 6, caractérisé en ce que la dépression réglée dans le volume intérieur du flacon est de préférence réglée à une valeur de 0,85 à 0,95 bar.